# EUROPEAN PATENT APPLICATION

(11) **EP 0 625 570 A1**
(43) Date of publication of application: **23.11.1994**
(21) Application number: 93303923.2
(22) Date of filing: 20.05.1993
(51) Int. Cl.: C12N 1/12

(54) **Process for the production of dried algal biomass**

(71) Applicant: NATIONAL RESEARCH DEVELOPMENT CORPORATION, New Delhi 110 048 (IN)
(72) Inventor: Seshadri, Chetpat Ventakasubban, Madras-600001, Tamil Nadu (IN); Umesh, Bangalore Venkatramu, Madras-600001, Tamil Nadu (IN)
(74) Representative: Green, Mark Charles

(57) **Abstract**

The invention relates to the production of dried algal biomass from spirulina, and consists in separate steps of culture growth. The first step is carried out in a reactor having a nutrient medium and receiving photons in the range of 1500 to 200 lux. Once the required optical density is achieved, the culture is subjected to a second step of culture growth in a nutrient and natural water. The initial optical density is 0.1 to 0.5. Once the desired optical density is achieved, the culture is harvested, filtered, washed and dried.

## Description

### BACKGROUND OF THE INVENTION

This invention relates to a process for the production of dried algal biomass from Spirulina.

### PRIOR ART

It is generally known that spirulina is a micro alga which contains valuable nutrients and is rich in proteins. Further, spirulina has an excellent vitamin complex which includes beta carotene (provitamin A) B₁,B₂,B₆, B₁₂,C,E and H (biotin) vitamins. Spirulina has also been shown to possess gama linolenic and alpha linolenic acids, which are very essential materials in combating diseases.

It is known that spirulina possesses various strains such as spirulina maxima, spirulina platensis, spirulina subsalsa and spirulina fusiformis. Strangely, most of the work hitherto conducted and reported in literature are on maxima and platensis-strains, and that very limited work has been conducted or reported in literature on spirulina fusiformis. Work on spirulina maxima has been reported in Appl. Microbiol Biotechnol (24,1, 47-50) 1986 Coden. Such a work is related to the mass culture of spirulina maxima in sea water, and with particular reference to the effect of nitrogen source on biomass yield. According to the report, spirulina maxima strain 4 MX was cultured outdoors from September 1984 to August 1985 using 6 PVC ponds, each with 3m² of illuminated surface. From July to August 1985, 3 concrete ponds of 10m² surface area were also used. Sea water was supplemented with a nutrient medium containing NaHCO₃, KNO₃ or urea, K₂HOP₄ or H₃PO₄ and Fe EDTA. The mean annual biomass yield using urea as the nitrogen source was substantially less in comparison to the standard bicarbanate medium. On sea water and nitrate, the yield was further reduced to 5.2 g/m²/day. Japanese patent no.61031095 discloses a process for the preparation of viscous polysaccharide from spirulina subsalsa. Such a process consists in inoculating spirulina subsala into a nutrient medium containing NaHCO₃, Mg SO₄,A5 solution, K₂HOP₄,CaCl₂, NaNO₃, Fe SO₄, NaCl, EDTA and wherein the A5 solution consisted of H₃BO₃, MnCl₂,ZnSO₄, 7H₂O and H₂SO₄. Cultivation was effected under 4000 lux of fluorescent light. The alga was collected and heated at 90°C in an aqueous solution containing NaCl and Na₂CO₃.

It is known to grow stock culture on agar slants using an alkaline nutrient medium containing nitrates and phosphates with a sub-culturing time of 30-40 days. Such a culture is transferred to glass carboys for further culturing using the same medium with a subculturing time of 30-40 days. The liquid stock cultures are made by separate sterilisation of the medium constituents and maintained for 30-40 days. Such liquid stock cultures are used for inoculum development with the same medium and kept under shaded conditions at 8000-10000 lux. These carboys are shaken a few times everyday before being used for outdoor ponds. Total time take in the process is about 90 days. Such a process can be utilised only on a batch wise manner and that the final product is produced after a considerable length of time, namely about 90 days.

### OBJECTS OF THE INVENTION

An object of the present invention is to propose an improved process for the production of dried algal biomass from spirulina obviating the disadvantages of the known processes.

Another object of the present invention is to propose a process for the production of dried algal biomass having the required quality and a yield of approximately 85% and, wherein, the time period is substantially reduced to, for example, 40 days.

### DETAILED DESCRIPTION OF THE INVENTION

According to the present invention there is provided a process for the production of dried algal biomass from spirulina which comprises in a first step of culturing spirulina in a first reactor having a nutrient containing sodium bicarbonate, sodium nitrate, dipotassium phosphate, trace elements such as zinc and vanadium in an aqueous medium and receiving photons in the range of 1500 to 2000 lux, to produce an innoculum having an optical density in the range of 0.8 to 1.2 measured at 420 namometers, subjecting the innoculum to a second step of culturing by introducing the innoculum into a second reactor being larger than the first reactor and having exposure to sunlight for receiving photons in the range of 4000-6000 lux, said second reactor having a nutrient and natural water, the initial optical density of the culture being in the range of 0.1 to 0.8 measured at 420 namomenter, harvesting the culture at an optical density of 1 to 1.2, filtering, washing and drying the culture to obtain the required algal biomass.

Strangely, it has now been found that an improved yield and a shorter processing period is obtained when spirulina strain is cultured in two separate reactors and, wherein, the first reactor receives photons in the range of 1500-2000 lux to produce a culture having an optical density in the range of 0.3 to 1.2 measured at 420 namometers. In the second reactor, the initial optical density of the culture is in the range of 0.1 to 0.5. The culture is harvested at an optical density of 1 to 1.2. The culture is filtered and the filterate and wash water is recirculated to the second reactor.

The specie of spirulina useful in the process of the present invention are spirulina maxima, spirulina plantensis and spirulina fusiformis. The process of preparing a culture in a pond, harvesting the culture, separating and washing the biomass from the growth medium, drying or subjecting the biomass to the step of dehydration are steps generally known in the art. However, the step of preparing the culture in two separate steps, the parameters and the method of performing each step is not known in the art.

The first step of culture growth consists in introducing an innoculum of spirulina into a first reactor containing a nutrient medium. Preferably, the ratio of the nutrient medium to innoculum is 3:1 to 4:1. The nutrient medium employed in the first step of culture growth consists of sodium bicarbonate, sodium nitrate, dipotassium phosphate and trace elements such as zinc and vanadium in an aqueous medium, such as distilled water.
Further, the culture receives photons in the range of 1500 to 2000 lux and at a temperature of 25 to 35°C. Such a treatment continues till the innoculum has an optical density of 0.8 to 1.2 measured at 420 namometers. However, the light absorption in the first reactor is selected at a wave length of 450 to 870 namometers. Normally, such an optical density is achieved within a period of 15 to 20 days.

The second step of culture growth consists in injecting the innoculum of the first step into a production pond or second reactor containing a nutrient medium similar to the first reactor except that it contains natural water, such as sea water. The reactors or ponds are designed such as to help the culture flow in shallow concentric channels. A culture depth of 20-30cm is advantageous for sustained production. Larger depth does not help significantly as penetration of light in the culture then becomes limited. Spirulina growth is monitored in terms of optical density measurements that are measured, for example, by a spectrophotometer.

The culture in the second reactor is subjected to photons in the range of 4000-6000 lux. The second reactor may be laid after levelling the ground, and can be as large as 60 meters long by 15 meters wide 30 cm deep and graded carefully so that smooth flow is ensured. The mass culture step entails daily monitoring of levels of N.P.O. and optical density in the culture to enable proper upkeep of the nutrients for growth of the culture. For example, a level flucuation of 40 mg/litre of nitrate nitrogen to 500 mg/litre of nitrate nitrogen on a daily basis may be inimical to good growth, whereas, fluctuation of 40-80 mg/litre might not be. Similarly nitrate nitrogen level should be kept down by redissolution and reabsorption into the culture, and which can be achieved by agitation of the innoculam. The concentration of nitrogen released from the sodium nitrate contained in the nutrient medium in the range of 40mg/litre to 500mg/litre is used in the second reactor. The required condition is also ensured by feeding the nutrients in the reactor by one shot feeding of the nutrients programmed methods or sinusoidal feeding and the like. The medium at the stage of culturing in the second reactor may contain between 500-3000 mg/litre of algal biomass.

The amount of sodium bicarbonate in the nutrient medium is by way of example approximately 10 gms/litre.

Agistation of culture during photosynthetic hours helps to increase the biomass growth. The level of agitation is expressed in terms of the velocity of flow achieved in the ponds. In general, a flow velocity of 20-40cm/sec is considered adequate for this purpose. Preferably, the agitation in the second reactor is effected at a speed of 5 to 25cm/sec. Constant agitation reduces the degree of super saturation of O₂ and nutritional gradients in the cells vicinity. The turbulence developed by agitation causes a favourable distribution of irradiance to cells growing in dense cultures when the light penetration becomes very limited.

Spirulina requires carbon for synthesis of carbohydrate, which is mainly supplied in the form of sodiumbicarbonate, and renders the growth medium alkaline thereby excluding other types of organisms growing in the medium. Normally in a pond, total alkalinity is maintained between 8-10g/l in the pond.

The algal culture is filtered by any suitable means, and the filtrate containing the nutrients is recycled into the second reactor or pond. By way of example, the washing is effected by a series of washing filters having a mesh size ranging from 50 to 400 mesh. The cake is washed by water spray and, whereby, it brings down the algal pH to near neutral and concentrates the slurry for the subsequent step of drying. The drying may be effected by spray drying at a temperature of 110 to 210°C.

The dried biomass prepared by the process of the invention for human consumption has the following charactertics :
Protein (Kjeldahl No. 6.25) 60-70%
c-phycocyanin 8.5-11%
Carbohydrates 14-16%
Lenolenic acid 3.9 and 28% lipids
Lipids 6-7% Vit B 20-60 mcg
Moisture 6-8%, Vit E, 7-8 IU
Total Carotenoids 200-250 mg/100gm
Calcium 600 mg/100 gm
Vitamin B₂ mg/100 gm
Phosphorocus oo mg/100 gm
Vitamin B₆ 1-2 mg/100 gm
Zinc 5-7 mg.100 gm
Iron 40 mg/100 gm
The present invention allows the algal to be cultured outdoors in natural sunlight using natural waters. The reactor sequence is in two stages as compared to the prior state of the art where more stages are used, thus effecting economics of operations.

By way of example and without implying any limitation thereto, the nutrient concentration to be maintained in the second reactor or pond is as follows :

| | |
|---|---|
| Nitrogen | 400 to 425 mg/lit |
| Potassium | 660 to 680 mg/lit |
| Phosphours | 80 to 100 mg/lit |
| Ca & Mg | 70 to 90 mg/lit as CaCo₃ |
| Sulphur | 160 to 190 mg/lit |
| Chloride | 550 to 650 mg/lit |
| Sodium | 5000 to 6500 mg/lit |

## Claims

1. A process for the production of dried algal biomass from spirulina which comprises in a first step of culturing spirulina in a first reactor having a nutrient containing sodium bicarbonate, sodium nitrate, dipotassium phosphate, trace elements such as zinc and vanadium in an aqueous medium and receiving photons in the range of 1500 to 2000 lux, to produce an innoculum having an optical density in the range of 0.8 to 1.2 measured at 420 namometers, subjecting the innoculum to a second step of culturing by introducing the innoculum into a second reactor being larger than the first reactor and having exposure to sunlight for receiving photons in the range of 4000-6000 lux, said second reactor having a nutrient and natural water, the initial optical density of the culture being in the range of 0.1 to 0.8 measured at 420 namometer, harvesting the culture at an optical density of 1 to 1.2, filtering, washing and drying the culture to obtain the required algal biomass.

2. A process as claimed in claim 1 wherein the first step of culture production is carried out at a temperature of 25 to 35°C.

3. A process as claimed in claims 1 & 2 wherein the amount of sodium bicarbonate in the nutrient medium is approximately 10 gms. per litre.

4. A process as claimed in claim 1-3 wherein the light absorption in the first reactor is selected at a wave length between 450-870 namometers.

5. A process as claimed in claim 1-4 wherein the concentration of nitrogen released from the sodium nitrate contained in the nutrient medium in the range of 40 mg/lt. to 500 mg/lit is used in the second reactor.

6. A process as claimed in claim 1-5 wherein the agitation of the innoculum in the second reactor is effected at a speed of 5-25 cm/sec.

7. A process as claimed in claim 1-6 wherein the washing is effected using a series of washing filters having a mesh-size ranging from 50 mesh - 400 mesh.

8. A process as claimed in claim 1-7 wherein the drying is effected at a temperature in the range of 110° - 210°C.
